# EUROPEAN PATENT SPECIFICATION

(11) **EP 1 644 005 B1**
(45) Date of publication and mention of the grant of the patent: **18.08.2010**
(21) Application number: 04743131.7
(22) Date of filing: 28.06.2004
(51) Int. Cl.: A61K 31/554, A61P 25/18

(54) **METABOLITE OF QUETIAPINE**
METABOLIT VON QUETIAPINE
METABOLITE DE QUETIAPINE

(30) Priority: 02.07.2003 US 484365 P
(43) Date of publication of application: 12.04.2006
(73) Proprietor: AstraZeneca AB, 151 85 Södertälje (SE)
(72) Inventor: GRIMM, Scott, W., AstraZeneca Wilmington, Wilmington, DE 19850-5437 (US); WINTER, Helen, R., AstraZeneca Wilmington, Wilmington, DE 19850-5437 (US); DAVIS,Patricia, C., AstraZeneca Wilmington, Wilmington, DE 19850-5437 (US); SUCKOW, Raymond F., West Patterson, NJ 07424 (US); GOLDSTEIN, Jeffrey, Wilmington, De 19850-5437 (US)
(74) Representative: Steinrud, Henrik
(86) International application number: PCT/GB2004/002783
(87) International publication number: WO 2005/002586

(56) References cited:
- US-A- 3 546 226
- DEVANE C LINDSAY ET AL: "Clinical pharmacokinetics of quetiapine: An atypical antipsychotic" CLINICAL PHARMACOKINETICS, vol. 40, no. 7, 2001, pages 509-522, XP009037259 ISSN: 0312-5963
- MUTSCHLER E: "Arzneimittelwirkungen" 1991, WISSENSCHAFTLICHE VERLAGSGESELLSCHAFT , STUTTGART , XP002298363 page 127, paragraph entitled "Indikationen"

## Description

### BACKGROUND OF THE INVENTION

The goal of antipsychotic drug development has been to develop agents with increased efficacy and safety along with fewer of the side effects commonly associated with the older antipsychotic medications. Quetiapine fumarate is described in U.S. Patent Number 4,879,288. Quetiapine fumarate is able to treat both the positive (hallucinations, delusions) and negative symptoms (emotional withdrawal, apathy) of psychosis and is associated with fewer neurological and endocrine related side effects compared to older agents. Quetiapine fumarate has also been associated with a reduction in hostility and aggression. Quetiapine fumarate is associated with fewer side effects such as EPS, acute dystonia, acute dyskinesia, as well as tardive dyskinesia. Quetiapine fumarate has also helped to, enhance patient compliance with treatment, ability to function and overall quality of life, while reducing recidivism. P. Weiden et al., *Atypical antipsychotic drugs and long-term outcome in schizophrenia*, 11 J. Clin. Psychiatry, 53-60, 57 (1996). Because of quetiapine fumarate's enhanced tolerability profile its use is particularly advantageous in the treatment of patients that are hypersensitive to the adverse effects of antipsychotic (such as elderly patients). Metabolites of quetiapine fumarate have been identified, E. Warawa et al. Behavioral approach to nondyskinetic dopamine antagonists: identification of Seroquel, 44 J. Med. Chem., 372 -389 (2001) and U.S. 4,879,288. One compound described in U.S. 4,879,288 is 11-piperazin-1-yldibenzo[*b*,*f*][1,4]thiazepine which has now been identified as a metabolite of quetiapine fumarate. 11-piperazin-1-yldibenzo[b,f][1,4]thiazepine has also been identified as a metabolite of quetiapine in Devane C Lindsay et al, Clin. Pharmacokinetics 40(7), 509-522 (2001).

### SUMMARY OF THE INVENTION

The invention is as defined in the appended claims.
11-piperazin-1-yldibenzo[*b*,*f*][1,4]thiazepine has the structure as shown by Formula I:

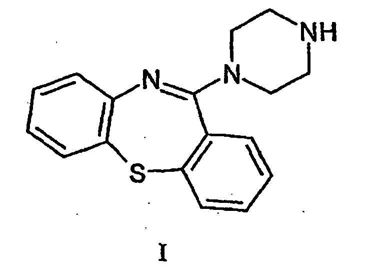

Provided herein is a pharmaceutical composition comprising the compound of Formula I or its pharmaceutically acceptable salt and at least one pharmaceutically acceptable carrier or a diluent. Further provided herein is the pharmaceutical composition for use in treating schizophrenia, dementia, anxiety, depression, mood disorders, bipolar disorders, bipolar mania, bipolar depression, cognitive disorders, psychosis, neurodegenerative disorders, agitation, hostility, panic, eating disorders, affective symptoms, mood symptoms and negative and positive psychotic symptoms. Further, provided herein is the use of the compound of Formula I and/or the pharmaceutical composition in the manufacture of a medicament for use in the treatment of the symptoms or conditions provided herein in a human.

### DETAILED DESCRIPTION OF THE INVENTION

The compound of Formula I is a dibenzothiazepine that has shown antidopaminergic activity. It has been shown to interact with a broad range of neurotransmitter receptors but has a higher affinity for serotonin (5-HT₂) receptors relative to dopamine (D₂) receptors in the brain. The compound of Formula I may be used as an antipsychotic with a reduction in the potential to cause side effects such as acute dystonia, acute dyskinesia, as well as tardive dyskinesia. Further the compound of Formula I may be used to treat patients of all ages and is advantageous in the treatment of elderly patients.

The term "mammal" means a warm-blooded animal, preferably a human.

The compound of Formula I may be made by a variety of methods known in the chemical arts. The compound of Formula I may be prepared by starting from known compounds or readily prepared intermediates including taking the lactam of Formula II: 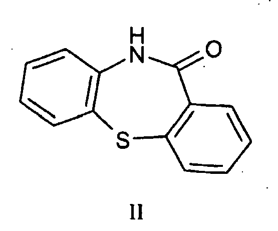 which may be prepared by methods well known in the literature, for example, as described by J. Schmutz et al. Helv. Chim. Acta., 48:336 (1965). The lactam of Formula II is treated with phosphorus chloride to generate the immino chloride of Formula III: 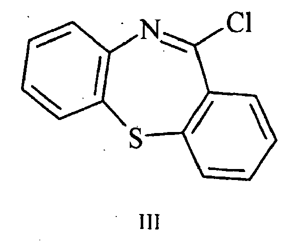 The immino chloride of Formula III may also be generated with other agents such as thionyl chloride or phosphorous pentachloride. The immino chloride is then reacted with piperazine to give the compound of Formula I.

The compound of Formula I provided herein is useful as a free base, but may also be provided in the form of a pharmaceutically acceptable salt, and/or in the form of a pharmaceutically acceptable hydrate. For example, pharmaceutically acceptable salts of Formula I include those derived from mineral acids such as for example: hydrochloric acid, nitric acid, phosphoric acid, sulfuric acid, hydroiodic acid, nitrous acid, and phosphorous acid, pharmaceutically acceptable salts may also be developed with organic acids including aliphatic mono dicarboxylates and aromatic acids. Other pharmaceutically acceptable salts of Formula I include but are not limited to hydrochloride, sulfate, pyrosulfate, bisulfate, bisulfite, nitrate, and phosphate.

A clinician may determine the effective amount by using numerous methods already known in the art, an example of which is the BPRS cluster score that can be used to assess levels of hostility and positive symptoms. The term "treating" within the context of the present invention encompasses to administer an effective amount of the compound of the present invention, to mitigate either a pre-existing disease state, acute or chronic, or a recurring symptom or condition. This definition also encompasses prophylactic therapies for prevention of recurring conditions and continued therapy for chronic disorders.

Particularly, the symptoms and conditions that may be treated by the administration of Formula I or its pharmaceutically acceptable salt or a pharmaceutical composition of Formula I, include but are not limited to anxiety, agitation, hostility, panic, eating disorders, affective symptoms, mood symptoms, negative and positive psychotic symptoms commonly associated with psychosis and neurodegenerative disorders.

Particular amount of the compound of Formula I or its pharmaceutically acceptable salt that may be administered in an amount up to 750 mg per day; the amount of the compound of Formula I or its pharmaceutically acceptable salt may be administered between 1 mg and 600 mg per day.

The compound of Formula I may be administered comprising a predetermined dosage of the compound of Formula I to a mammal between one and four times a day, wherein the predetermined dosage is between 1 mg and 600 mg.

The compound of Formula I may be administered at an initial predetermined dosage to a human patient twice a day, wherein the predetermined dosage is between 1 mg and 30 mg with increases in increments of 1-50 mg twice daily on the second and third day as tolerated. Thereafter, further dosage adjustments can be made at intervals of no less than 2 days.

In one embodiment of the invention the pharmaceutical composition comprises up to 750 mg of the compound of Formula I or its pharmaceutically acceptable salt thereof per day.

In another embodiment of the invention, the pharmaceutical composition may comprise between 100 mg and 400 mg per day of the compound of Formula I or a pharmaceutically acceptable salt thereof.

The pharmaceutical composition of the invention may accordingly be obtained by conventional procedures using conventional pharmaceutical excipients. Thus, pharmaceutical compositions intended for oral use may contain, for example, one or more coloring, sweetening, flavoring and/or preservative agents.

For preparing pharmaceutical compositions from the compound of Formula I of this invention, inert, pharmaceutically acceptable carriers can be either solid or liquid. Solid form preparations include powders, tablets, dispersible granules, capsules, cachets and suppositories.

The amount of active ingredient that is combined with one or more excipients to produce a single dosage form will necessarily vary depending upon the host treated. The size of the dose for therapeutic or prophylactic purposes of a compound of the Formula I will naturally vary according to the nature and severity of the symptoms or conditions and the age and sex of the animal or patient, according to well known principles of medicine.

In a further aspect, the present invention provides the use of a compound of Formula I, or a pharmaceutically acceptable salt thereof, in the manufacture of a medicament for use in treating the symptoms or conditions provided herein.

In a further aspect, the composition of the invention can be used for treating at least one symptom or condition associated with schizophrenia, dementia, anxiety, depression, mood disorders, bipolar disorders, bipolar mania, bipolar depression, cognitive disorders, psychosis and neurodegenerative disorders by administering to a mammal an effective amount of the compound of Formula I or its pharmaceutically acceptable salt and one or more of other therapeutically active agents, benzodiazepines, 5-HT_{1A} ligands, 5-HT_{1B} ligands, 5-HT_{1D} ligands, mGluR2A agonists, mGluR5 antagonists, antipsychotics, NKl receptor antagonists, antidepressants, or serotonin reuptake inhibitors administered in combination as part of the same pharmaceutical composition, or administered separately as part of an appropriate dose regimen designed to obtain the benefits of combination therapy. The appropriate dose regimen, the amount of each dose of an active agent administered, and the specific intervals between doses of each active agent will depend upon the subject being treated, the specific active agent being administered and the nature and severity of the specific disorder or condition being treated. In general, the compounds of this invention, when used as either a single active agent or when used in combination with another active agent, will be administered to a subject in an amount up to about 750 mg per day, in single or divided doses. Such compounds may be administered on a regimen of up to 6 times per day, preferably 1 to 4 times per day. Variations may nevertheless occur depending upon the subject being treated and the individual response to the treatment, as well as on the type of pharmaceutical formulation chosen and the time period and interval at which such administration is carried out. In some instances, dosage levels below the lower limit of the aforesaid range may be more than adequate, while in other cases larger doses may be employed to achieve the desired effect, provided that such larger doses are first divided into several small doses for administration throughout the day.

Exemplary benzodiazepines may include but are not limited to adinazolam, alprazolam, bromazepam, clonazepam, chlorazepate, chlordiazepoxide, diazepam, estazolam, flurazepam, balezepam, lorazepam, midazolam, nitrazepam, oxazepam, quazepam, temazepam, triazolam and equivalents thereof.

Exemplary 5-HT_{1A} and/or 5HT_{1B} ligands may include but are not limited to buspirone, alnespirone, elzasonan, ipsapirone, gepirone, zopiclone and equivalents thereof.

Exemplary mGluR 2 agonists may include (1S,3R)-1-aminocyclopentane-1,3-dicarboxylic acid, (2S,3S,4S)alpha-(carboxycyclopropyl)glycine, and 3,5-dihydroxyphenylglycine.

Exemplary antidepressants may include but are not limited to maprotiline, amitriptyline, clomipramine, desipramine, doxepin, imipramine, nortryptyline, protriptyline, trimipramine, SSRIs and SNRIs such as fluoxetine, paroxetine, citalopram, escitalopram, sertraline, venlafaxine, fluoxamine, and reboxetine.

Exemplary antipsychotics may include but are not limited to clozapine, risperidone, quetiapine, olanzapine, amisulpride, sulpiride, zotepine, chlorpromazine, haloperidol, ziprasidone, and sertindole.

The following examples provided are not meant to limit the invention in any manner and are intended for illustrative purposes only.

### EXAMPLES

### Example 1

### Preparation of 11-piperazin-1-yldibenzo[b,f][1,4]thiazepine

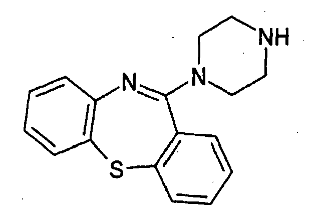

Into a 1000ml round-bottom flask equipped with a magnetic stirring bar and reflux condenser with a nitrogen inlet was charged with 25.0 grams (g) (0.110 mole) of dibenzo[b,f][1,4]thiazepine-11(10-H)-one (made by the method disclosed by J. Schmutz et al. Helv. Chim. Acta., 48: 336 (1965)), as a dry solid, followed by 310ml POCl₃ and 3ml of N,N-dimethylaniline. The reaction mixture was heated at reflux (106 degrees C) for 6 hours giving a clear orange solution. The reaction was then cooled to room temperature, and POCl₃ removed on the rotary evaporator leaving an orange oil. This residue was partitioned between ice -water (500ml) and Ethyl acetate (800ml). The layers were separated and the aqueous phase extracted with Ethyl acetate (3 X 200ml). The combined Ethyl acetate extracts were dried over MgSO₄, filtered, and then stripped down on the rotary evaporator, leaving the crude Imino chloride as a light yellow solid (26.26g, 97% yield). The structure was confirmed by NMR and Mass Spectrum (300MHz, CDCl₃; ES+, M+1 = 246.7). Crude Imino chloride (27.35g, 0.111 mole) was added to 1000ml o-xylene in a 2000ml round-bottom flask equipped with a magnetic stir bar and a reflux condenser with nitrogen inlet. To this solution was added commercially available piperazine (47.95g, 0.557 mole) in one portion as a dry solid at room temperature. The mixture was stirred until nearly all the piperazine dissolved. Then the reaction mixture was heated at reflux (142 degrees C) for 40 hours (out of convenience). The reaction was then allowed to cool to room temperature, and an aliquot was partitioned between 1N NaOH / CH₂Cl₂. The organic phase was checked by TLC (silica gel, CH₂Cl₂ / Methanol 90:10, lodoplatinate visualized) and showed clean conversion to one major product (Rf = 0.45). A drop of the reaction solution was diluted with CH₃CN to prepare a sample for LC / MS analysis, which confirmed the presence of the desired product (M+1 = 296.4). The reaction mixture was stripped down on the rotary evaporator under high vacuum to remove the xylene. The residue was partitioned between 1N NaOH (400ml) and CH₂Cl₂ (200ml). The layers were separated, and the aqueous phase further extracted with CH₂Cl₂ (3 X 200ml). The combined CH₂Cl₂ extracts were washed with brine (200ml), then dried over MgSO₄, filtered, and stripped down on the rotary evaporator to give the crude title compound as a yellow gum (35.3g). The crude free base was purified by flash column chromatography over silica gel (600g) eluting with a gradient of 0 to 20% Methanol in CH₂Cl₂. Fractions containing the pure desired product were combined and stripped down on the rotary evaporator, to afford the purified free base as a light yellow foam (25.67g, 78% yield).

### Example 2

### Preparation of 11-piperazin-1-yldibenzo[b,f][1,4]thiazepine, dihydrochloride salt

The free base was converted to it's dihydrochloride salt by dissolving it in a mixture of Methanol (125ml) and Diethyl ether (125ml), then treating with 250ml of 1.0 M HCl/ Ether (Aldrich). An off -white gummy solid separated initially, and the mixture was further diluted with 500ml ether. The gummy solid did not solidify on prolonged stirring. The solvents were decanted away from the gum. The gum was treated with absolute Ethanol (200ml), then stirred until crystallization occurred, giving a thick white suspension of crystals. This mixture was then slowly diluted with ether (800ml) and allowed to stir overnight to complete the crystallization. The dihydrochloride salt was isolated by filtration, washed with ether (3 X 50ml), then dried in vacuum at 60 degrees C to afford the dihydrochloride salt of the title compound as a white solid (31.64g, 98.8% conversion).

### ANALYSIS:

The product was characterized by NMR and LC / MS (300MHz, CDCl₃; AP+, M+1 = 296.4).

### Example 3 (mice assays)

An assessment of dopamine antagonism was made in rodent models. The methods and procedures used can be found in J. Med. Chem., 44 (3), 372 -389, 2001. The results are as follows the binding affinity for brain serotonin 5-HT₂ receptor was 27 K 1 nM, and for dopamine D₁ and D₂ receptors was 1489 and 234 K1 nM, respectively. These results show that the compound of the present invention as the dihydrochloride salt interacts with a broad range of neurotransmitter receptors, however, the assay also reveals that that the compound of the present invention as the dihydrochloride salt has a higher affinity for serotonin (5-HT₂) receptors relative to dopamine (D₂) receptors in the brain. It is this combination of serotonin and dopamine receptor antagonism, with higher relative 5-HT₂ to D₂ receptor affinity that indicates the compound of Formula I as a potent atypical antipsychotic. J. Goldstein, Quetiapine Fumarate (Seroquel): a new atypical antipsychotic, 35(3) Drugs of Today 193-210 (1999).

## Claims

1. A pharmaceutical composition comprising an effective amount of 11-piperazin-1-yldibenzo[b,*f*][1,4]thiazepine having the structure as shown by Formula I: 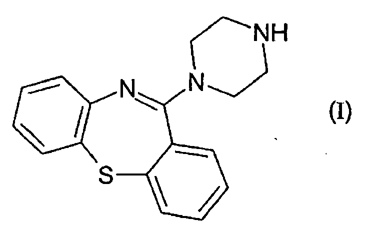 or a pharmaceutically acceptable salt thereof together with at least one pharmaceutically acceptable carrier or diluent.

2. The composition as recited in Claim 1 wherein 11-piperazine-1-yldibenzo[b,*f*][1,4]thiazepine comprises a free base.

3. The composition as recited in Claim 1, wherein the composition is an oral pharmaceutical composition.

4. The composition as recited in Claim 1 wherein the amount of 11-piperazin-1-yldibenzo[b,*f*][1,4]thiazepine or its pharmaceutically acceptable salt comprises up to 750 mg.

5. The composition as recited in Claim 4 wherein the amount of 11-piperazin-1-yldibenzo[b,*f*][1,4]thiazepine or its pharmaceutically acceptable salt comprises between 1 to 600 mg.

6. The composition as recited in Claim 5 wherein the amount of 11-piperazin-1-yldibenzo[b,*f*][1,4]thiazepine or its pharmaceutically acceptable salt comprises between 100 to 400 mg.

7. A composition as defined in claim 1 or 2 for use in treating schizophrenia, dementia, anxiety, depression, mood disorders, bipolar disorders, bipolar mania, bipolar depression, cognitive disorders, psychosis, neurodegenerative disorders, agitation, hostility, panic, eating disorders, affective symptoms, mood symptoms and negative and positive psychotic symptoms.

8. The composition as recited in Claim 7 wherein the composition is for use in treating schizophrenia.

9. The composition as recited in Claim 7 wherein the composition is for use in treating psychosis.

10. The composition as recited in Claim 7 wherein the composition is for use in treating bipolar depression.

11. The composition as recited in Claim 7 wherein the composition is for use in treating bipolar mania.

12. The composition as recited in Claim 7 wherein the composition is for use in treating anxiety.

13. The composition as recited in Claim 7 wherein the composition is for use in treating a mood disorder.

14. The composition as recited in Claim 7 wherein the composition is for use in treating depression.

15. Use of 11-piperazin-1-yldibenzo[b,*f*][1,4]thiazepine or a pharmaceutically acceptable salt thereof or the pharmaceutical composition of claim 1 or 2 in the manufacture of a medicament for the treatment of schizophrenia, dementia, anxiety, depression, mood disorders, bipolar disorders, bipolar mania, bipolar depression, cognitive disorders, psychosis, neurodegenerative disorders, agitation, hostility, panic, eating disorders, affective symptoms, mood symptoms and negative and positive psychotic symptoms in a human.

16. The use as recited in claim 15 wherein the medicament is for the treatment of schizophrenia.

17. The use as recited in claim 15 wherein the medicament is for the treatment of psychosis.

18. The use as recited in claim 15 wherein the medicament is for the treatment of bipolar depression.

19. The use as recited in claim 15 wherein the medicament is for the treatment of bipolar mania.

20. The use as recited in claim 15 wherein the medicament is for the treatment of anxiety.

21. The use as recited in claim 15 wherein the medicament is for the treatment of depression.

22. The use as recited in claim 15 wherein the medicament is for the treatment of a mood disorder.

23. The use as recited in Claim15wherein the amount of 11-piperazin-1-yldibenzo[b,*f*][1,4]thiazepine or its pharmaceutically acceptable salt is up to 750 mg per day.

24. The use as recited in Claim 23 wherein the amount of 11-piperazin-1-yldibenzo[b,*f*][1,4]thiazepine or its pharmaceutically acceptable salt is between 1 to 600 mg per day.

25. The use as recited in Claim 24 wherein the amount of 11-piperazin-1-yldibenzo[b,*f*][1,4]thiazepine or its pharmaceutically acceptable salt is between 100 to 400 mg per day.

26. The use as recited in claim 15, wherein the medicament is for oral administration.

27. The use as recited in Claims 15, 16, 17, 18, 19, 20, 21, 22, 23, 24 25 or 26 wherein the 11-piperazin-1-yldibenzo[b,*f*][1,4]thiazepine comprises a free base.

## Patentansprüche

1. Pharmazeutische Zusammensetzung, enthaltend eine wirksame Menge an 11-Piperazin-1-yldibenzo[b,f][1,4]thiazepin mit der wie in Formel I gezeigten Struktur: 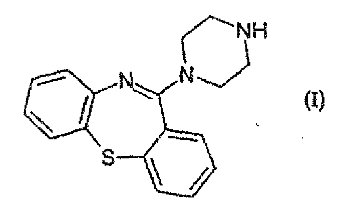 oder einem pharmazeutisch annehmbaren Salz davon zusammen mit wenigstens einem pharmazeutisch annehmbaren Träger oder Verdünnungsmittel.

2. Zusammensetzung nach Anspruch 1, wobei 11-Piperazin-1-yldibenzo[b,*f*][1,4]thiazepin eine freie Base umfaßt.

3. Zusammensetzung nach Anspruch 1, wobei es sich bei der Zusammensetzung um eine orale pharmazeutische Zusammensetzung handelt.

4. Zusammensetzung nach Anspruch 1, wobei die Menge an 11-Piperazin-1-yldibenzo[b,*f*][1,4]thiazepin oder seinem pharmazeutisch annehmbaren Salz bis zu 750 mg umfaßt.

5. Zusammensetzung nach Anspruch 4, wobei die Menge an 11-Piperazin-1-yldibenzo[b,*f*][1,4]thiazepin oder seinem pharmazeutisch annehmbaren Salz 1 bis 600 mg umfaßt.

6. Zusammensetzung nach Anspruch 5, wobei die Menge an 11-Piperazin-1-yldibenzo[b,*f*][1,4]thiazepin oder seinem pharmazeutisch annehmbaren Salz 100 bis 400 mg umfaßt.

7. Zusammensetzung nach Anspruch 1 oder 2 zur Verwendung bei der Behandlung von Schizophrenie, Demenz, Angst, Depression, affektiven Psychosen, bipolaren Störungen, bipolarer Manie, bipolarer Depression, kognitiven Störungen, Psychose, neurodegenerativen Störungen, Erregtheit, Feindseligkeit, Panik, Eßstörungen, affektiven Symptomen, Gemütssymptomen und negativen und positiven psychotischen Symptomen.

8. Zusammensetzung nach Anspruch 7, wobei die Zusammensetzung für die Verwendung bei der Behandlung von Schizophrenie bestimmt ist.

9. Zusammensetzung nach Anspruch 7, wobei die Zusammensetzung für die Verwendung bei der Behandlung von Psychose bestimmt ist.

10. Zusammensetzung nach Anspruch 7, wobei die Zusammensetzung für die Verwendung bei der Behandlung von bipolarer Depression bestimmt ist.

11. Zusammensetzung nach Anspruch 7, wobei die Zusammensetzung für die Verwendung bei der Behandlung von bipolarer Manie bestimmt ist.

12. Zusammensetzung nach Anspruch 7, wobei die Zusammensetzung für die Verwendung bei der Behandlung von Angst bestimmt ist.

13. Zusammensetzung nach Anspruch 7, wobei die Zusammensetzung für die Verwendung bei der Behandlung von einer affektiven Psychose bestimmt ist.

14. Zusammensetzung nach Anspruch 7, wobei die Zusammensetzung für die Verwendung bei der Behandlung von Depression bestimmt ist.

15. Verwendung von 11-Piperazin-1-yldibenzo [b, f] [1,4]thiazepin oder eines pharmazeutisch annehmbaren Salzes davon oder der pharmazeutischen Zusammensetzung nach Anspruch 1 oder 2 bei der Herstellung eines Medikaments zur Behandlung von Schizophrenie, Demenz, Angst, Depression, affektiven Psychosen, bipolaren Störungen, bipolarer Manie, bipolarer Depression, kognitiven Störungen, Psychose, neurodegenerativen Störungen, Erregtheit, Feindseligkeit, Panik, Eßstörungen, affektiven Symptomen, Gemütssymptomen und negativen und positiven psychotischen Symptomen bei einem Menschen.

16. Verwendung nach Anspruch 15, wobei das Medikament für die Behandlung von Schizophrenie bestimmt ist.

17. Verwendung nach Anspruch 15, wobei das Medikament für die Behandlung von Psychose bestimmt ist.

18. Verwendung nach Anspruch 15, wobei das Medikament für die Behandlung von bipolarer Depression bestimmt ist.

19. Verwendung nach Anspruch 15, wobei das Medikament für die Behandlung von bipolarer Manie bestimmt ist.

20. Verwendung nach Anspruch 15, wobei das Medikament für die Behandlung von Angst bestimmt ist.

21. Verwendung nach Anspruch 15, wobei das Medikament für die Behandlung von Depression bestimmt ist.

22. Verwendung nach Anspruch 15, wobei das Medikament für die Behandlung von einer affektiven Psychose bestimmt ist.

23. Verwendung nach Anspruch 15, wobei die Menge an 11-Piperazin-1-yldibenzo[b,*f*][1,4]thiazepin oder seinem pharmazeutisch annehmbaren Salz bis zu 750 mg pro Tag beträgt.

24. Verwendung nach Anspruch 23, wobei die Menge an 11-Piperazin-1-yldibenzo[b,*f*][1,4]thiazepin oder seinem pharmazeutisch annehmbaren Salz 1 bis 600 mg pro Tag beträgt.

25. Verwendung nach Anspruch 24, wobei die Menge an 11-Piperazin-1-yldibenzo[b,*f*][1,4]thiazepin oder seinem pharmazeutisch annehmbaren Salz 100 bis 400 mg pro Tag beträgt.

26. Verwendung nach Anspruch 15, wobei das Medikament für die orale Verabreichung bestimmt ist.

27. Verwendung nach einem der Ansprüche 15, 16, 17, 18, 19, 20, 21, 22, 23, 24, 25 oder 26, wobei das 11-Piperazin-1-yldibenzo[b,*f*][1,4]thiazepin eine freie Base umfaßt.

## Revendications

1. Composition pharmaceutique comprenant une quantité efficace de la 11-pipérazin-1-yldibenzo[b,f][1,4]thiazépine ayant la structure représentée par la formule I : 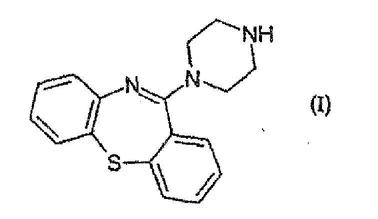 ou un sel pharmaceutiquement acceptable de celle-ci, conjointement avec au moins un support ou diluant pharmaceutiquement acceptable.

2. Composition selon la revendication 1, **caractérisée en ce que** la 11-pipérazin-1-yldibenzo[b,f][1,4]thiazépine comprend une base libre.

3. Composition selon la revendication 1, **caractérisée en ce que** la composition est une composition pharmaceutique orale.

4. Composition selon la revendication 1, **caractérisée en ce que** la quantité de 11-pipérazin-1-yldibenzo[b,f][1,4]thiazépine ou de son sel pharmaceutiquement acceptable comprend jusqu'à 750 mg.

5. Composition selon la revendication 4, **caractérisée en ce que** la quantité de 11-pipérazin-1-yldibenzo[b,f][1,4]thiazépine ou de son sel pharmaceutiquement acceptable comprend entre 1 et 600 mg.

6. Composition selon la revendication 5, **caractérisée en ce que** la quantité de 11-pipérazin-1-yldibenzo[b,f][1,4]thiazépine ou de son sel pharmaceutiquement acceptable comprend entre 100 et 400 mg.

7. Composition telle que définie dans la revendication 1 ou 2, destinée à être utilisée dans le traitement de la schizophrénie, d'une démence, de l'anxiété, de la dépression, des troubles de l'humeur, des troubles bipolaires, de la manie bipolaire, de la dépression bipolaire, des troubles cognitifs, d'une psychose, des troubles neurodégénératifs, de l'agitation, de l'hostilité, d'une panique, de troubles du comportement alimentaire, des symptômes affectifs, des symptômes de l'humeur et des symptômes psychotiques négatifs et positifs.

8. Composition selon la revendication 7, **caractérisée en ce que** la composition est destinée à être utilisée dans le traitement de la schizophrénie.

9. Composition selon la revendication 7, **caractérisée en ce que** la composition est destinée à être utilisée dans le traitement d'une psychose.

10. Composition selon la revendication 7, **caractérisée en ce que** la composition est destinée à être utilisée dans le traitement de la dépression bipolaire.

11. Composition selon la revendication 7, **caractérisée en ce que** la composition est destinée à être utilisée dans le traitement de la manie bipolaire.

12. Composition selon la revendication 7, **caractérisée en ce que** la composition est destinée à être utilisée dans le traitement de l'anxiété.

13. Composition selon la revendication 7, **caractérisée en ce que** la composition est destinée à être utilisée dans le traitement d'un trouble de l'humeur

14. Composition selon la revendication 7, **caractérisée en ce que** la composition est destinée à être utilisée dans le traitement de la dépression.

15. Utilisation de la 11-pipérazin-1-yldibenzo[b,*f*][1,4]thiazépine ou d'un sel pharmaceutiquement acceptable de celle-ci ou de la composition pharmaceutique selon la revendication 1 ou 2, dans la fabrication d'un médicament destiné au traitement de la schizophrénie, d'une démence, de l'anxiété, de la dépression, des troubles de l'humeur, des troubles bipolaires, de la manie bipolaire, de la dépression bipolaire, des troubles cognitifs, d'une psychose, des troubles neurodégénératifs, de l'agitation, de l'hostilité, d'une panique, de troubles du comportement alimentaire, des symptômes affectifs, des symptômes de l'humeur et des symptômes psychotiques négatifs et positifs chez l'homme.

16. Utilisation selon la revendication 15, **caractérisée en ce que** le médicament est destiné au traitement de la schizophrénie.

17. Utilisation selon la revendication 15, **caractérisée en ce que** le médicament est destiné au traitement d'une psychose.

18. Utilisation selon la revendication 15, **caractérisée en ce que** le médicament est destiné au traitement de la dépression bipolaire.

19. Utilisation selon la revendication 15, **caractérisée en ce que** le médicament est destiné au traitement de la manie bipolaire.

20. Utilisation selon la revendication 15, **caractérisée en ce que** le médicament est destiné au traitement de l'anxiété.

21. Utilisation selon la revendication 15, **caractérisée en ce que** le médicament est destiné au traitement de la dépression.

22. Utilisation selon la revendication 15, **caractérisée en ce que** le médicament est destiné au traitement d'un trouble de l'humeur.

23. Utilisation selon la revendication 15, **caractérisée en ce que** la quantité de 11-pipérazin-1-yldibenzo[b,*f*][1,4]thiazépine ou de son sel pharmaceutiquement acceptable est jusqu'à 750 mg par jour.

24. Utilisation selon la revendication 23, **caractérisée en ce que** la quantité de 11-pipérazin-1-yldibenzo[b,*f*][1,4]thiazépine ou de son sel pharmaceutiquement acceptable est comprise entre 1 et 600 mg par jour.

25. Utilisation selon la revendication 24, **caractérisée en ce que** la quantité de 11-pipérazin-1-yldibenzo[b,*f*][1,4]thiazépine ou de son sel pharmaceutiquement acceptable est comprise entre 100 et 400 mg par jour.

26. Utilisation selon la revendication 15, **caractérisée en ce que** le médicament est destiné à l'administration par voie orale.

27. Utilisation selon les revendications 15, 16, 17, 18, 19, 20, 21, 22, 23, 24, 25 ou 26, **caractérisée en ce que** la 11-pipérazin-1-yldibenzo[b,f][1,4]thiazépine comprend une base libre.
